# EUROPEAN PATENT SPECIFICATION

(11) **EP 2 197 397 B1**
(45) Date of publication and mention of the grant of the patent: **16.04.2014**
(21) Application number: 08828579.6
(22) Date of filing: 02.09.2008
(51) Int. Cl.: A61F 5/02

(54) **ADJUSTABLE BACK SUPPORT DEVICE**
VERSTELLBARE RÜCKENSTÜTZVORRICHTUNG
DISPOSITIF DE SOUTIEN DORSAL AJUSTABLE

(30) Priority: 30.08.2007 US 968954 P; 02.09.2008 US 202863
(43) Date of publication of application: 23.06.2010
(73) Proprietor: Toso, Victor, St. Paul, Minnesota 55108 (US)
(72) Inventor: Toso, Victor, St. Paul, Minnesota 55108 (US)
(74) Representative: Curley, Donnacha John
(86) International application number: PCT/US2008/075040
(87) International publication number: WO 2009/029941

(56) References cited:
- EP-A- 0 421 045
- US-A- 5 643 184
- US-A- 5 645 080
- US-A- 6 083 183
- US-B1- 6 202 236

## Description

### Field

The present invention relates generally to back support devices. More specifically, the present invention pertains to adjustable back support devices for supporting a lower back region of an individual in a seated position.

### Background

Many activities require participants to be seated in an upright position for an extended length of time without rigid back support. Examples of recreational activities which require an individual to be seated upright for extended periods of time include canoeing, fishing, hunting, boating, horseback riding, and motorcycling. Other activities such as working at a computer may also require an individual to be seated in an upright position for extended periods of time. Many people find sitting with little or no back support to be very uncomfortable, in some cases distracting from their participation in these activities. Such discomfort may also cause the individual to reduce the amount of time they spend engaged in those activities. For example, on a long motorcycle journey, frequent stops may be required because of discomfort to the lower back. The frequency and severity of discomfort may be increased or exacerbated due to improper posture and/or a weak back structure.

A number of different back support devices have been developed which provide support to a user's lower back. The ability to easily adjust these devices to provide a desired fit or to accommodate for variations in body size is often limited, however, preventing the user from comfortably wearing the device for extended periods of time. In some designs, for example, the back support device may not permit the user to adjust the size of the device in order to give the user a more relaxed fit and/or to improve body posture. In such case, the inability to adjust the device may limit its use. A need therefore exists for back support devices that can be easily adjusted to accommodate for the user's preferences as well as specific body size.

US 6,083,183 discloses a waistband device comprising a rectangular cushion and two knee straps. A V-shaped connecting band member is used to connect the cushion to one end of each knee strap. The second end of each knee strap is provided with a fastener which allows the second ends of the knee straps to be coupled together.

US 5,643,184 discloses a back support with foot engaging straps comprising knee straps. The straps are attached to the back support member at a single point each by an intervening buckle and a short strap.

US 5,643,080 is directed towards a waist supported carrying case which includes a back support. The ends of the knee straps are attached to each other, and the straps are integrally attached within a central sleeve of the waist support.

US 6,202,236 is directed towards a shooting harness for supporting a user's upper back.

EP-A-0 421 045 discloses all the features of claim 1 with the exception of the features that the knee straps are elastic and that a back support adjustment mechanism is provided for adjusting the length of the back support member.

### Brief Summary

The present invention relates to devices for supporting a lower back region of an individual in a seated position. A back support device in accordance with an illustrative embodiment can include a flexible, double-ended back support member adapted to provide lumbar support to the user's lower back, and a number of elastic knee straps extending from the ends of the back support member that form several loops that can be attached to the user's knees when the user is in a seated position. The back support member can be constructed from a flexible material that supports the user's lower back when worn. In some embodiments, for example, the flexible material can include elastic webbing formed integrally with or as a separate component from the material forming the elastic knee straps. The elastic webbing can have a strength sufficient to provide back support to the user without the use of additional padding or cushions, although such additional support can be provided, if desired. In some embodiments, the back support member can be made from multiple layers of elastic webbing to increase the amount of back support provided to the user's back.

An adjustment mechanism can be used to adjust the length of the elastic knee straps, allowing the user to vary the size of the loops to provide a more relaxed fit and/or to improve body posture. In one such embodiment, for example, the adjustment mechanism can include a fabric hook and loop type fastener coupled to the ends of the back support member and to the ends of the elastic knee straps. A fastener mechanism can also be provided in some embodiments to permit the user to secure the elastic knee straps together at a location between the first and second ends thereof. When used, the fastener mechanism helps to support the user's knees together while also preventing the loops from inadvertently detaching during use.

A back support adjustment mechanism is provided to permit the user to adjust the length of the back support member. The back support adjustment mechanism can include, for example, a fabric hook-type fastener attached to a portion of the back support member, which can be configured to mate with several fabric receptor flaps coupled to the ends of the back support member. In use, the length of the back support member can be adjusted by releasing the fabric receptor flaps and then repositioning the flaps relative to the fabric hook to either decrease or increase the length of the back support member, as desired.

The back support member may further define an interior pouch that permits the back support device to be folded upon itself and collapsed for storage when not in use. A number of carrying handles can be provided within the interior pouch to facilitate carrying the back support device once stored within the interior pouch. In some embodiments, the interior pouch may be further configured to receive a therapeutic device such as a thermal pack or vibration element. The interior pouch can be formed from an unsewn interior portion of the back support member, creating an opening through which the therapeutic device can be inserted into the back support member.

### Brief Description of the Drawings

Figure 1 is a perspective view of a back support device in accordance with an illustrative embodiment having an adjustment mechanism for adjusting several elastic knee straps;
Figure 2 is another perspective view showing the illustrative back support member of Figure 1;
Figure 3 is an enlarged perspective view showing the adjustment mechanism for one of the elastic knee straps of Figure 2;
Figure 4 is a perspective view showing the illustrative back support device of Figure 1 attached to a user;
Figure 5 is a perspective view of a back support device in accordance with another illustrative embodiment having a back support adjustment mechanism;
Figure 6 is an enlarged perspective view showing the back support adjustment mechanism of Figure 5 in greater detail;
Figure 7 is another enlarged perspective view of the back support adjustment mechanism of Figure 5 showing the flap detached from the fabric hook;
Figure 8 is a perspective view showing the rear of the back support device of Figure 5 attached to a user's body;
Figure 9 is a perspective view of a back support device in accordance with another illustrative embodiment having an interior pouch;
Figure 10 is a perspective view showing an interior pouch of the back support device of Figure 9;
Figure 11 is a perspective view showing the opening of the interior pouch in a fully expanded position; and
Figure 12 is a perspective view showing the back support device of Figure 9 folded upon itself within the interior pouch.

### Detailed Description

The following description should be read with reference to the drawings, in which like elements in different drawings are numbered in like fashion. The drawings, which are not necessarily to scale, depict selected embodiments and are not intended to limit the scope of the invention. Although examples of construction, dimensions, and materials are illustrated for the various elements, those skilled in the art will recognize that many of the examples provided have suitable alternatives that may be utilized.

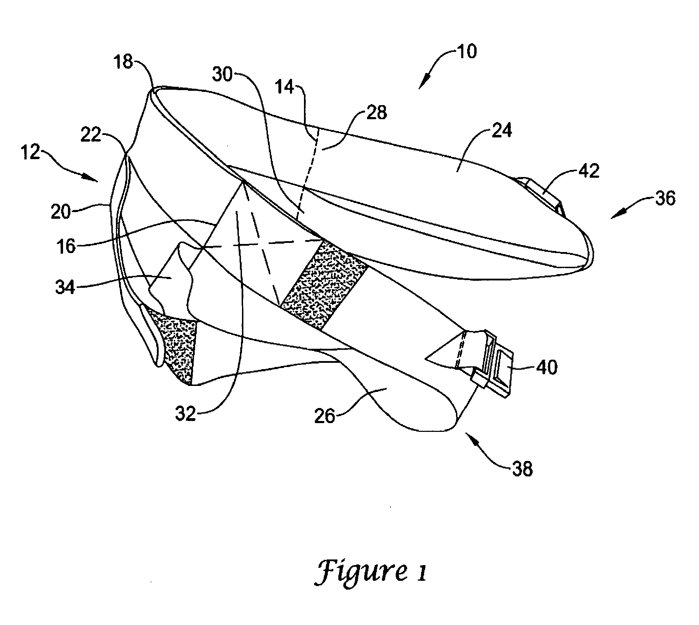

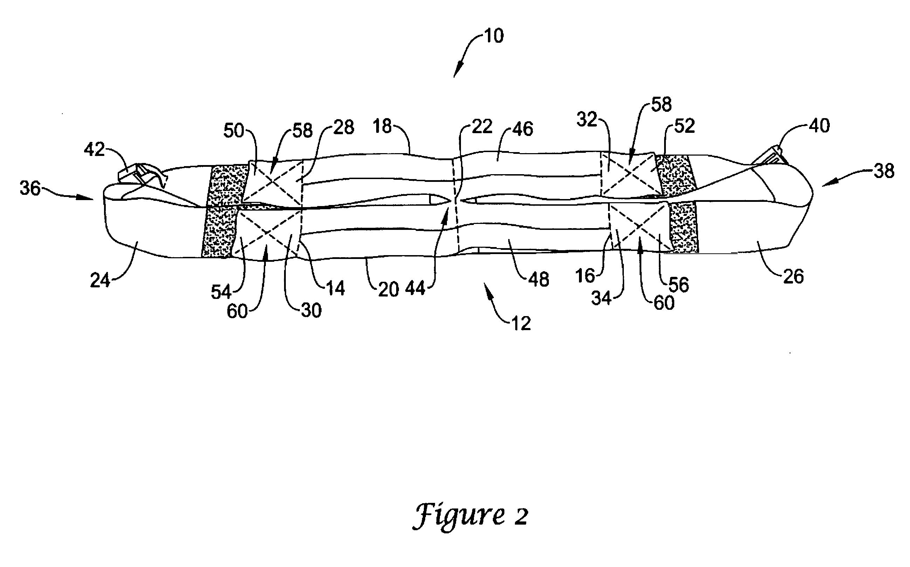

Referring now to Figures 1 and 2, a back support device 10 in accordance with an illustrative embodiment having an adjustment mechanism for adjusting several elastic knee straps will now be described. In the illustrative embodiment depicted, the back support device 10 includes a back support member 12 having an elongated and generally rectangular shape adapted to fit about and provide lombar support for the lower portion of a user's back. The back support member 12 can have a double-ended structure including a first end 14, a second end 16, an upper side 18, and a lower side 20. The width of the back support member 12 may be defined generally by the length between the upper 18 and the lower side 20, and can be configured to span substantially the length of the lumbar region of the user's back. The length of the back support member 12, in turn, may be defined generally by the length between the first end 14 and second end 16, and can be configured to span the circumference of the user's lower back.

The length and/or width dimensions of the back support member 12 may vary depending on the size of the user, the amount of back support desired, as well as other factors. An example length of the back support member 12 can be from about 25,4 cm (10 inches) to about 40,6 cm (16 inches), and more specifically about 30.5 cm (12 inches) to about 35,6 cm (14 inches). An example width of the back support member 12 can be from about 5,08 cm (2 inches) to 20,3 cm (8 inches), and more specifically about 10,2 cm (4 inches) to 15,2 cm (6 inches). Other length and/or width dimensions are possible, however. In addition, while the illustrative back support member 12 depicted in Figure 1 has a substantially rectangular shape, it should be understood that the shape of the member 12 may vary from that shown. In some embodiments, for example, the width of the back support member 12 may be greater towards the center portion 22 of the member 12 than at the ends 14,16 to provide additional support to the center portion of the user's lower back, if desired.

The back support member 12 can be fabricated from a flexible material adapted to support the user's lower back when worn. An example of a flexible material can include elastic webbing such as that commonly used in the construction of back support devices. Other materials such as canvass may also be utilized. In some embodiments, the back support member 12 may be cushioned or padded, although versions where no additional cushioning or padding is provided are also contemplated. The material forming the back support member 12 can be folded upon itself one or more times and then sewn together along the ends 14,16 and sides 18,20, forming a multi-layered structure. In those embodiments where elastic webbing is used, for example, the webbing material can be folded upon itself one or more times and then sewn together along the ends 14,16 and/or sides 18,20 to form a multi-layered structure that increases the amount of back support provided to the user's back.

The back support device 10 may further include a number of elastic knee straps 24,26 extending from each end 14,16 of the back support member 12. A first (*i.e*. left) elastic knee strap 24 extending from the first end 14 end of the back support member 12, for example, can include a first end 28 located adjacent to an upper left portion of the back support member 12, and a second end 30 located adjacent to a lower left portion of the back support member 12. A second (i.e. right) elastic knee strap 26 extending from the second end 16 of the back support member 12, in turn, can include a first end 32 located adjacent to an upper right portion of the back support member 12, and second end 34 located adjacent to a lower right portion of the back support member 12. The first and second elastic knee straps 24,26 can each have an elongated shape having a length of about 63,5 - 88,9 cm (25 to 35 inches), and more specifically, about 76,2 cm (30 inches), although other lengths are possible.

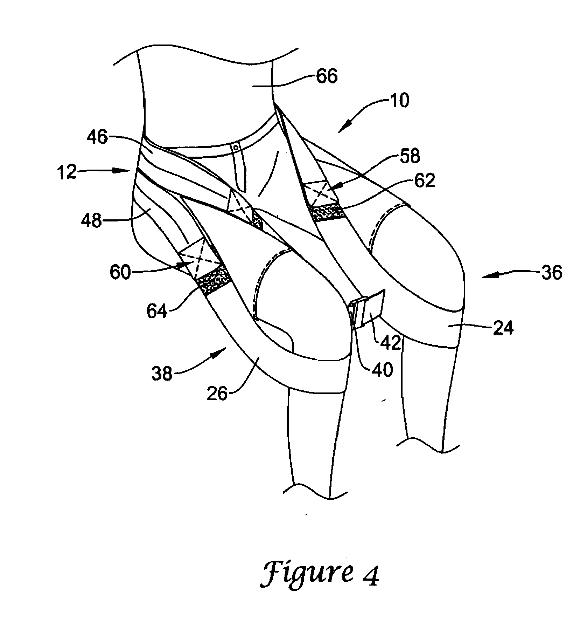

The first and second elastic knee straps 24,26 may be formed integrally as a continuous piece with the material forming the back support member 12, or can each comprise separate members that are attached to the ends 14,16 of the support member 12 by sewing, adhesion, or other suitable attachment means. For example, the first and second elastic knee straps 24,26 may be formed integrally with the elastic webbing or other such material used to construct the back support member 12. In use, and as discussed further with respect to Figure 4, each of the elastic knee straps 24,26 can be configured to form a respective loop 36,38 that can be attached at a location approximately midway along their length to the user's knees when the user is in a seated position. If desired, the elastic knee straps 24,26 can include a padded knee brace (not shown) for even disbursement of pressure for the user's knees when the device 10 is used in a seated position.

Each of the elastic knee straps 24,26 can be connected together at a location approximately midway along their length via a fastener. In some embodiments, and as shown in Figure 1, the fastener can include a male buckle member 40 coupled to the second elastic knee strap 26 and adapted to engage a corresponding female buckle member 42 coupled to the first elastic knee strap 24. Other fastener mechanisms such as a fabric hook and latch-type fastener (*e.g*. a VELCRO fastener), a clasp, and/or a drawstring or tie could also be utilized to releasably secure the loops 36,38 together. When connected, the buckle members 40,42 can be used to prevent the loops 36,38 from inadvertently slipping off of the user's knees during use. In addition, the buckle members 40,42 help to maintain the spacing of the user's legs when the device 10 is being used. In certain embodiments, one or both of the buckle members 40,42 can be made adjustable, allowing the user to adjust the separation or distance between the loops 36,38 in order to provide more or less lateral support to the user's legs.

Figure 2 is another perspective view showing the illustrative back support member 12 of Figure 1. As shown in Figure 2, an interior side 44 of the back support member 12 adapted to cradle all or a portion of the user's lower back can include a number of elastic straps 46,48, which provide lumbar support to the user's lower back while also permitting the adjustment of the elastic knee straps 24,26. An upper elastic strap 46 of the back support member 12 having a first end 50 and a second end 52, for example, may extend lengthwise along the upper periphery of the back support member 12, and can be used to independently adjust the lengths of the elastic knee straps 24,26. In similar fashion, a lower elastic strap 48 of the back support member 12 having a first end 54 and a second end 56 can also be used to independently adjust the length of the elastic knee straps 24,26. The upper and lower elastic straps 46,48 can be fabricated from elastic webbing similar to that used in the construction of the elastic knee straps 24,26. In certain embodiments, for example, the upper and lower elastic straps 46,48 can include 5,08 or 7,62 cm (2 or 3-inch) wide elastic webbing that is sewn together.

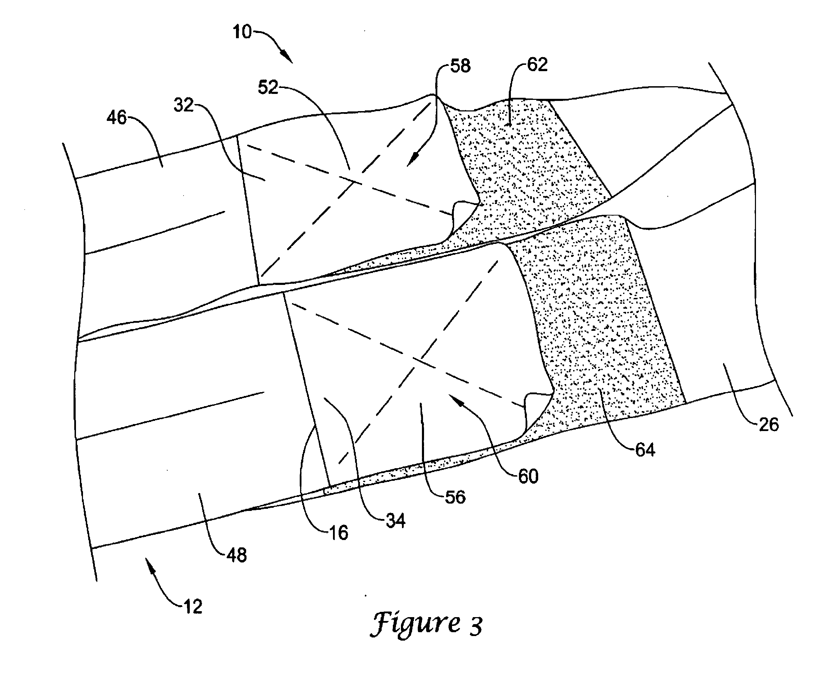

Each end 50,52 of the upper and lower elastic straps 46,48 can include an adjustment mechanism 58,60 that can be utilized to adjust the length of the elastic knee straps 24,26. As can be seen in further detail in Figure 3, for example, the second end 52 of the upper elastic strap 46 can be made from a fabric hook (e.g. VELCRO) or the like, and can be configured to secure to a corresponding fabric receptor surface 62 formed on the end 32 of the second elastic knee strap 26. In similar fashion, the second end 56 of the lower elastic strap 48 can be made from a fabric hook or the like, and can be configured to secure to a corresponding fabric receptor surface 64 formed on the other end 34 of the second elastic knee strap 26. If desired, other types of adjustment mechanisms such as a tri-slide buckle could also be used to adjust the length of the elastic knee straps 24,26. In use, each of the adjustment mechanisms 58,60 can be used to adjust the length of the elastic knee strap 26 to the size and/or desired fit of the user. A similar set of adjustment mechanisms (not shown) can be provided on the opposite ends 50,54 of the upper and lower elastic straps 46,48 to adjust the length of the left elastic knee strap 24, as desired.

Figure 4 is a perspective view showing a user 66 wearing the back support device 10 of Figure 1. As shown in Figure 4, the back support member 12 is adapted to fit against the lumbar region of the user's back with the elastic knee straps 24,26 looped over the user's knees when the user is in a seated position. When worn in this manner, the force of the user's knees pulls the back support member 12 against the lumbar region of the user's lower back, forming an elastic chair that provides back support to the user while in the seated position. In this position, the buckle members 40,42 may or may not be connected, as desired. When connected, the buckle members 40,42 maintain the knees of the user 66 together while also preventing the loops 36,38 from inadvertently detaching during use.

To vary the amount of back support provided by the device 10, or to adjust the device 10 to fit different sized users, the length of the elastic knee straps 24,26 can be adjusted using the adjustment mechanisms 58,60. To adjust the length of the right knee strap 26, for example, the user can remove one or both of the fabric hooks on the ends 52,56 of the upper and lower elastic straps 46,48 and then reposition the hooks on the fabric receptors 62,64 to either increase or decrease the length of the elastic knee strap 26, as desired. Adjustment of the left elastic knee strap 24 can be accomplished in similar fashion via the adjustment mechanisms coupled to the ends of the elastic knee strap 24.

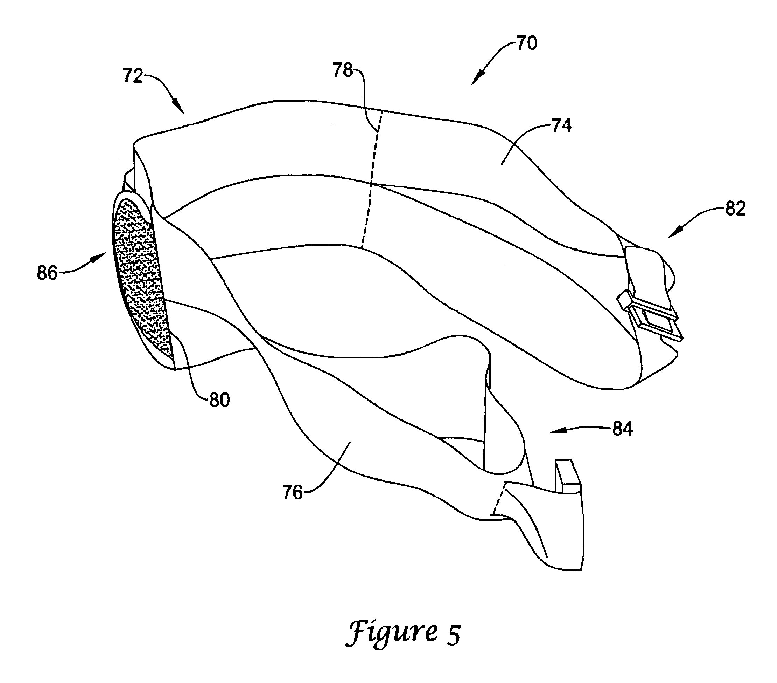

Figure 5 is a perspective view of a back support device 70 in accordance with another illustrative embodiment having a back support adjustment mechanism. Back support device 70 is similar to the back support device 10 described above with respect to Figures 1-4, including a flexible, double-ended back support member 72 configured to fit adjacent to the user's lower back, and a number of elastic knee straps 74,76 extending from each end 78,80 of the back support member 72. The elastic knee straps 74,76 can each be configured to form a respective loop 82,84 that can be attached at a location approximately midway along their length to the user's knees when the user is in a sitting position, forming an elastic chair that provides back support for the lumbar region of the user's lower back.

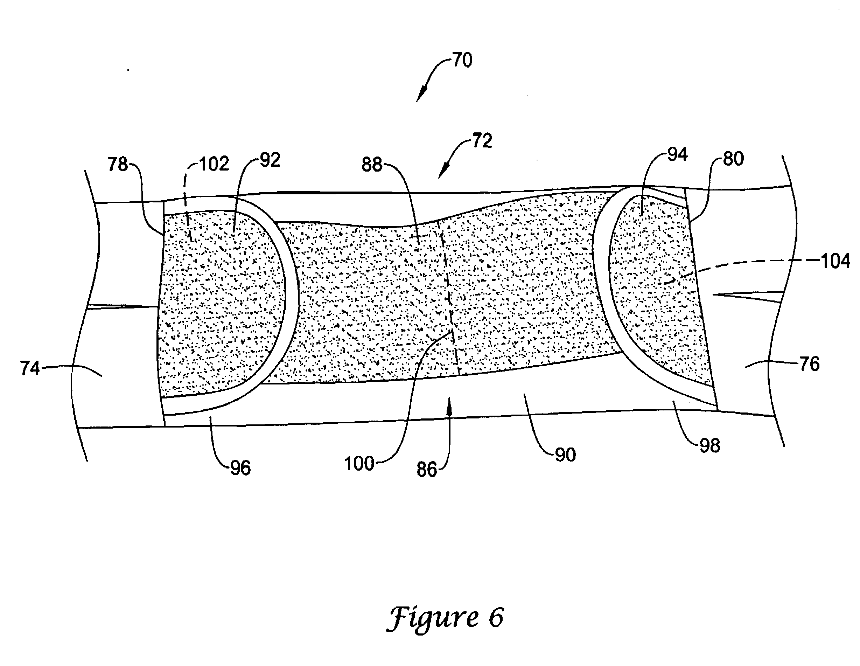

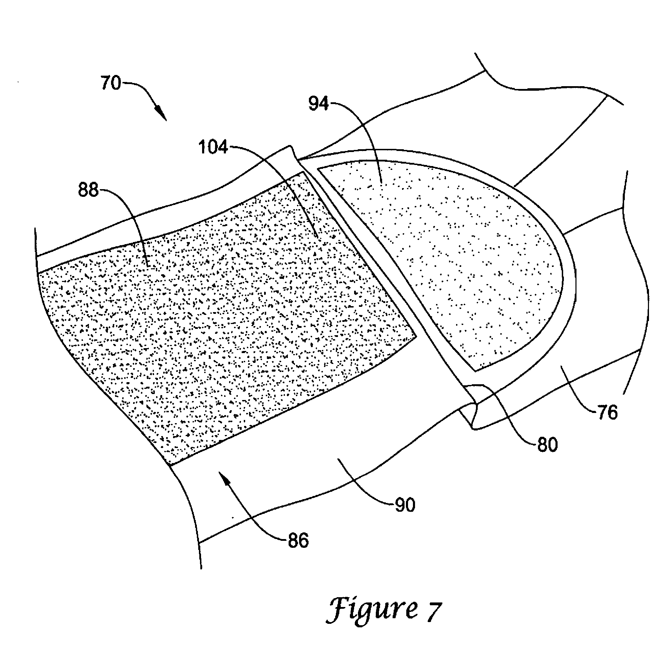

In the illustrative embodiment, the back support device 70 further includes a back support adjustment mechanism 86 that can be used to adjust the length of the back support member 72. As can be further seen in Figure 6, for example, the adjustment mechanism 86 can include a fabric hook 88 attached to the interior side 90 of the back support member 72 and adapted to mate with several fabric receptor flaps 92,94 coupled to the ends 96,98 of the member 72. The fabric hook 88 can be secured to the center portion of the back support member 72 along a stitching line 100, and can include a pair of free ends 102,104 that are configured to mate with and receive the fabric receptor flaps 92,94. As can be seen further in Figure 7, the underside portion of the fabric receptor flaps 92,94 can be fabricated from a material such as VELCRO that can be releasably secured to the surface of the fabric hook 88, allowing the flaps 92,94 to be releasably secured to the hook 88.

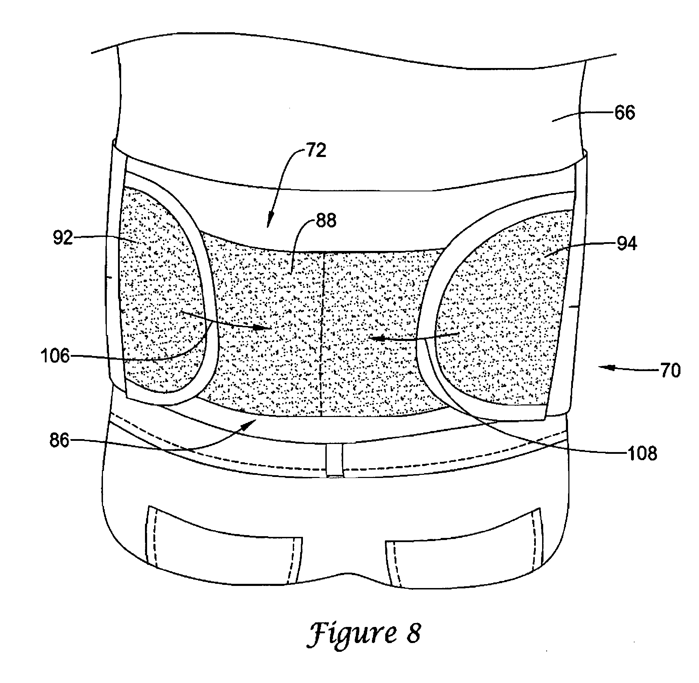

Figure 8 is a perspective view showing the rear of the back support device 70 of Figure 5 attached to a user's 66 body. As shown in Figure 8, the length of the back support member 72 can be adjusted by releasing the fabric receptor flaps 92,94 from the fabric hook 88, and then either moving the flaps 92,94 either closer to or further away from the center portion of the fabric hook 88 depending on whether the user desires to decrease or increase the length of the back support member 72. If, for example, the user desires to decrease the length of the back support member 72, the user can pull the fabric receptor flaps 92,94 inwardly in the direction indicated generally by arrows 106,108, causing the length of the member 72 to shorten. By adjusting the fabric receptor flaps 92,94 relative to the fabric hook 88 in this manner, the back support member 72 can be adjusted to a number of different sizes to accommodate the size and/or wearing preferences of the user.

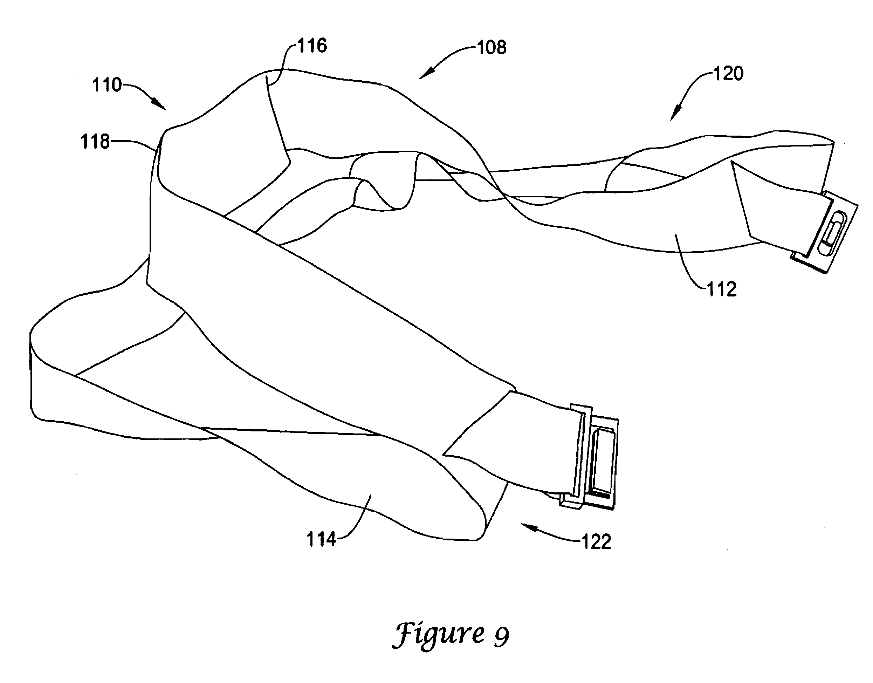

Figure 9 is a perspective view of a back support device 108 in accordance with another illustrative embodiment having an interior pouch. The back support device 108 can be configured similar to the other back support devices 10,70 described herein, including a back support member 110 configured to fit adjacent to the user's lower back, and a number of elastic knee straps 112,114 extending from each end 116,118 of the back support member 110 and forming a respective loop 120,122 that can be attached to the user's knees.

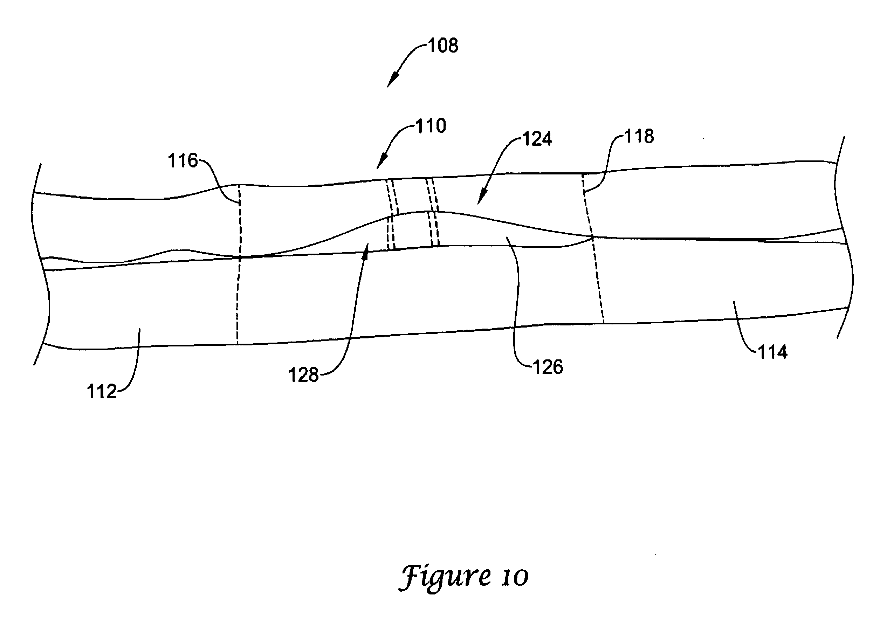

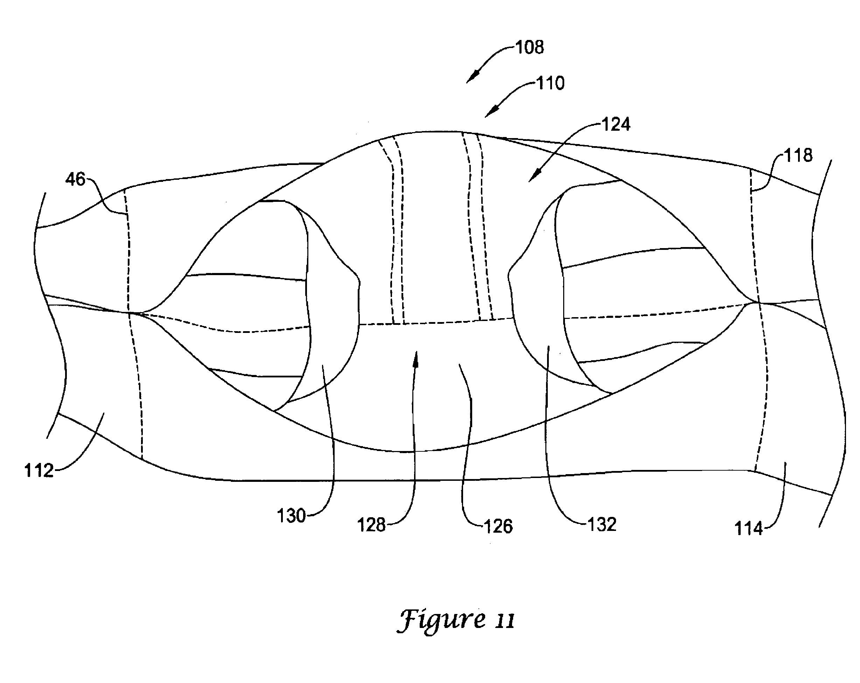

Figure 10 is a perspective view showing an interior pouch 124 of the back support device 108 of Figure 9. As shown in Figure 10, the interior pouch 124 may define a cavity 126 that can be used to fold the back support device 108 upon itself when not in use, and for receiving a therapeutic device such as a thermal pack or vibration element when the device 108 is worn. In certain embodiments, the interior pouch 124 may be formed from an unsewn portion of the back support member 110, forming an opening 128 that can be used to place the therapeutic device within the cavity 126. When the back support member 110 is wrapped around the user's lower back, the opening 128 can be configured to close similar to a coin purse, holding the therapeutic device securely in place within the cavity. As shown in another view in Figure 11 with the opening 128 fully expanded, a number of handles 130,132 within the interior pouch 124 can be provided to facilitate carrying of the back support device 108 once the device 108 is folded upon itself and collapsed.

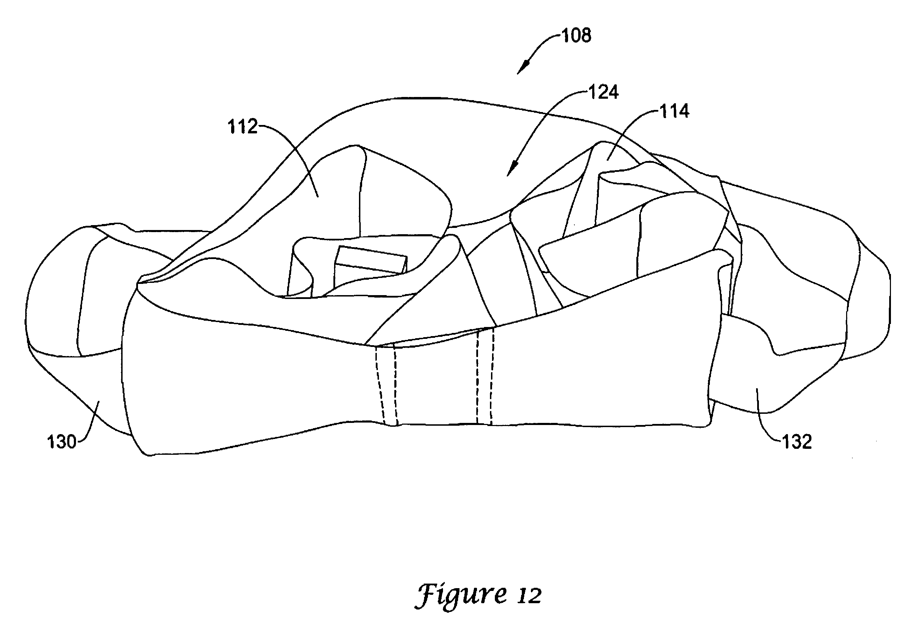

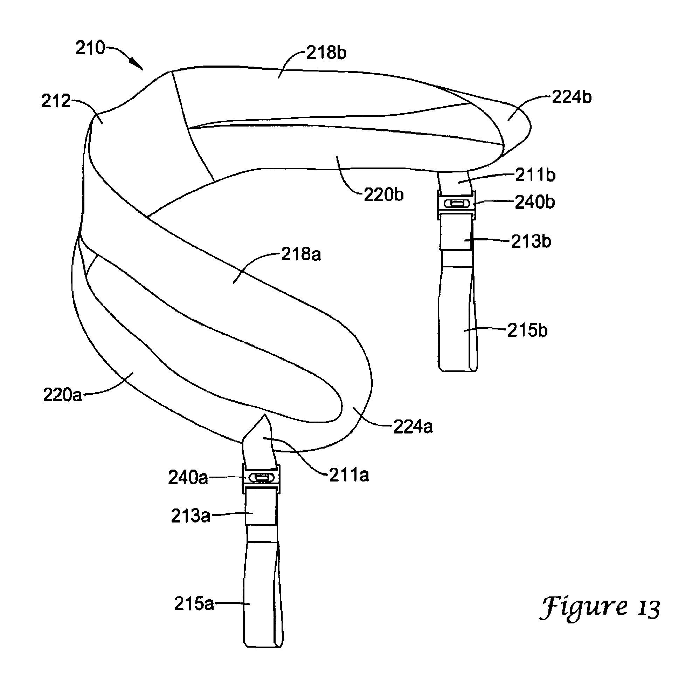

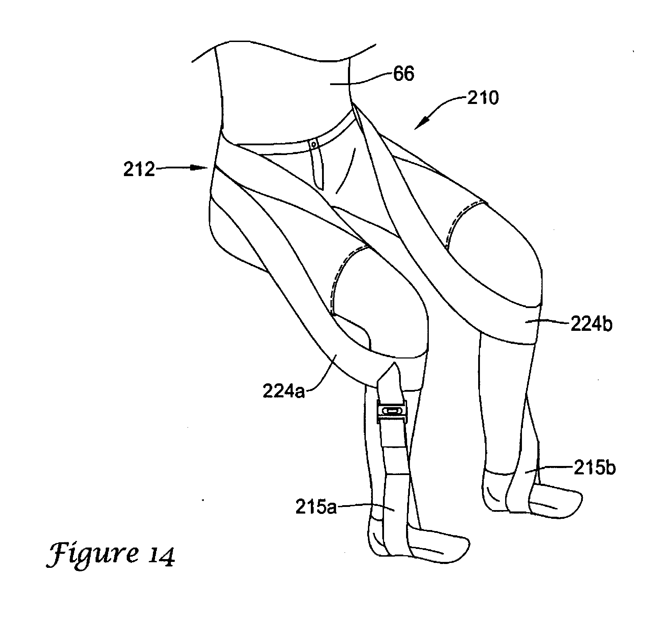

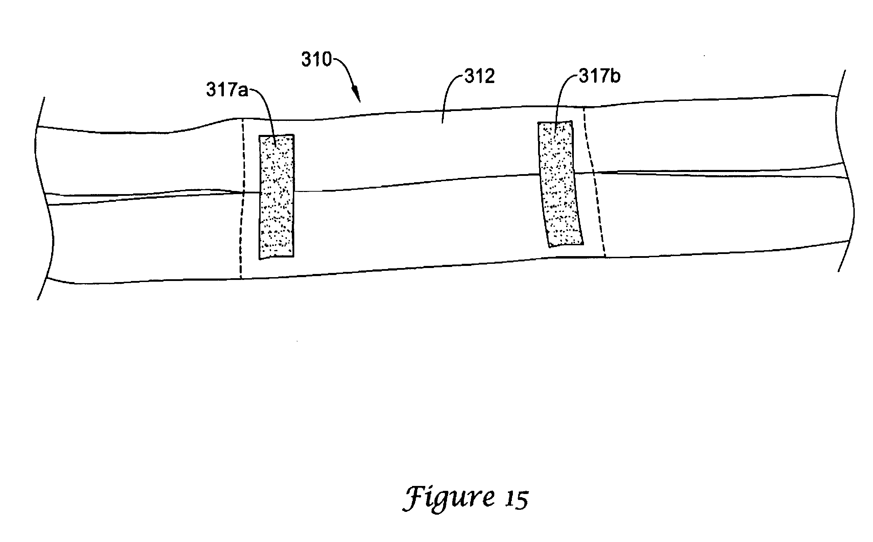

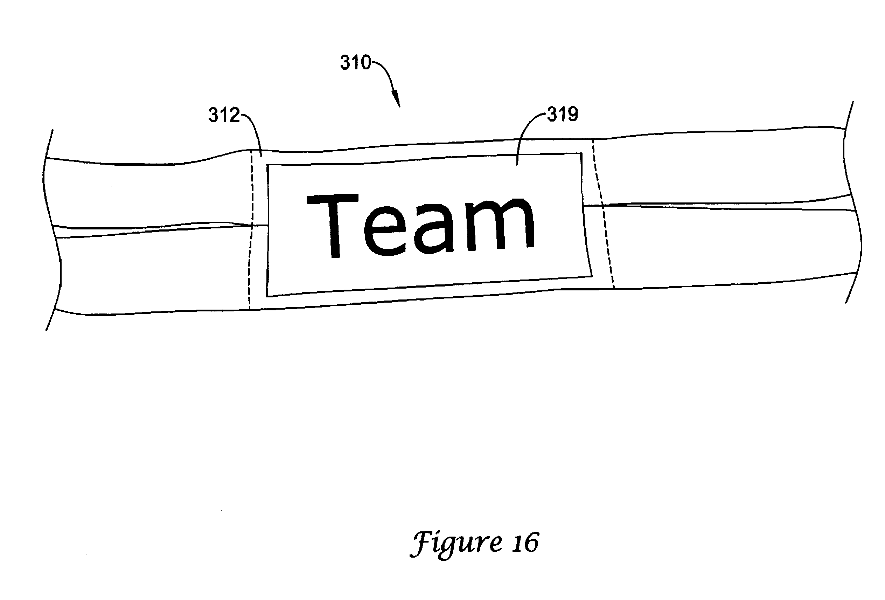

Figure 12 is a perspective view showing the back support device 110 of Figure 9 partially folded upon itself and collapsed within the interior pouch 124. As shown in Figure 11, the elastic knee straps 112,114 can be folded into the interior pouch 124 for storage or transport. In this position, the handles 130,132 secured to the back support member 110 can be used to help carry the device 108.

While several different features are described herein with respect to specific embodiments depicted in the drawings, it is contemplated that the back support device can include any number of different combinations of features. For example, the back support device can include both an adjustment mechanism for adjusting the length of the elastic knee straps and a back support adjustment mechanism for adjusting the length of the back support member.

Having thus described the several embodiments of the present invention, those of skill in the art will readily appreciate that other embodiments may be made and used which fall within the scope of the claims attached hereto. Numerous advantages of the invention covered by this document have been set forth in the foregoing description. It will be understood that this disclosure is, in many respects, only illustrative. Changes may be made in details, particularly in matters of shape, size and arrangement of parts without exceeding the scope of the invention.

## Claims

1. A back support device (10) for supporting a user's lower back, comprising:
a double-ended back support member (12) adapted to provide lumbar support to the user's lower back, wherein the back support comprises a first end (14) and a second end (16) and a length between the first end and the second end;
a plurality of elastic knee straps (24, 26) extending from the ends of the back support member, each of said plurality of elastic knee straps including a first end (28, 32) and a second end (30, 34) both coupled to or formed integrally with an end of the back support member (12) and forming a loop (36, 38) adapted to engage a knee of the user when the user is in a seated position;
an adjustment mechanism (58, 60) for selectively adjusting the lengths of each of the elastic knee straps (24, 26); and
a fastener (40, 42) for securing the elastic knee straps (24, 26) together at a location between said first and second ends of the elastic knee straps (24, 26); and
a back support adjustment mechanism (86) for adjusting the length of the back support member.

2. The back support device (10) of claim 1, wherein the back support member includes a flexible fabric material.

3. The back support device (10) of claim 2, wherein the flexible fabric material comprises elastic webbing.

4. The back support device (10) of claim 3, wherein the elastic webbing includes a plurality of webbing layers.

5. The back support device (10) of any preceding claim, wherein said plurality of elastic knee straps (24, 26) each comprise elastic webbing.

6. The back support device (10) of any preceding claim, wherein the adjustment mechanism includes a fabric hook adapted to engage a fabric receptor patch.

7. The back support device (10) of any preceding claim, wherein the back support adjustment mechanism (58, 60) includes a fabric hook adapted to engage a number of fabric receptor flaps (62,64).

8. The back support device (108) of any preceding claim, wherein the back support member (110) further defines an interior pouch (124) adapted to permit the back support device (108) to be folded upon itself and stored therein.

9. The back support device (108) of any preceding claim, wherein the back support member (110) further includes a number of carrying handles (130, 132).

10. The back support device (108) of claim 8, further comprising a therapeutic device disposed within the interior pouch (124).

11. The back support device (10) of any preceding claim, wherein the back support device (10) is an elastic chair.

12. The back support device (10) of claim 1, wherein the fastener is coupled to the elastic knee straps (24, 26) at a location midway along their length between said first (28, 32) and second (30, 34) ends.

## Patentansprüche

1. Rückenstützvorrichtung (10) zum Stützen des unteren Rückens eines Benutzers, umfassend:
ein zweiendiges Rückenstützteil (12), das dazu angepasst ist, eine Lumbalstütze für den unteren Rücken des Benutzers bereitzustellen, wobei die Rückenstütze ein erstes Ende (14) und ein zweites Ende (16) sowie ein Stück zwischen dem ersten Ende und dem zweiten Ende umfasst;
mehrere elastische Kniegurte (24, 26), die sich von den Enden des Rückenstützteils aus erstrecken, wobei jeder der mehreren elastischen Kniegurte ein erstes Ende (28, 32) und ein zweites Ende (30, 34) beinhaltet, die beide gekoppelt sind an oder einstückig ausgebildet sind mit ein(em) Ende des Rückenstützteils (12) und eine Schlinge (36, 38) bilden, die dazu angepasst ist, an einem Knie des Benutzers anzugreifen, wenn sich der Benutzer in einer Sitzposition befindet;
einen Regulierungsmechanismus (58, 60) zum Regulieren der Längen jedes der elastischen Kniegurte (24, 26) nach Wahl; und
einen Verschluss (40, 42) zum Sichern der elastischen Kniegurte (24, 26) aneinander an einer Stelle zwischen dem ersten und dem zweiten Ende der elastischen Kniegurte (24, 26); und
einen Rückenstützen-Regulierungsmechanismus (86) zum Regulieren der Länge des Rückenstützteils.

2. Rückenstützvorrichtung (10) nach Anspruch 1, wobei das Rückenstützteil ein flexibles Gewebematerial beinhaltet.

3. Rückenstützvorrichtung (10) nach Anspruch 2, wobei das flexible Gewebematerial elastisches Gurtband umfasst.

4. Rückenstützvorrichtung (10) nach Anspruch 3, wobei das elastische Gurtband mehrere Gurtbandlagen beinhaltet.

5. Rückenstützvorrichtung (10) nach einem der vorstehenden Ansprüche, wobei die mehreren elastischen Kniegurte (24, 26) jeweils elastisches Gurtband umfassen.

6. Rückenstützvorrichtung (10) nach einem der vorstehenden Ansprüche, wobei der Regulierungsmechanismus ein Gewebehakenteil beinhaltet, das dazu angepasst ist, eine Gewebeaufnehmerstelle zu greifen.

7. Rückenstützvorrichtung (10) nach einem der vorstehenden Ansprüche, wobei der Rückenstützen-Regulierungsmechanismus (58, 60) ein Gewebehakenteil beinhaltet, das dazu angepasst ist, eine Anzahl von Gewebeaufnehmerklappen (62, 64) zu greifen.

8. Rückenstützvorrichtung (108) nach einem der vorstehenden Ansprüche, wobei das Rückenstützteil (110) weiterhin einen Innenbeutel (124) begrenzt, der angepasst ist, um zu ermöglichen, dass die Rückenstützvorrichtung (108) auf sich selbst gefaltet und in demselben aufbewahrt wird.

9. Rückenstützvorrichtung (108) nach einem der vorstehenden Ansprüche, wobei das Rückenstützteil (110) weiterhin eine Anzahl von Traggriffen (130, 132) beinhaltet.

10. Rückenstützvorrichtung (108) nach Anspruch 8, die weiterhin eine innerhalb des Innenbeutels (124) angeordnete therapeutische Vorrichtung umfasst.

11. Rückenstützvorrichtung (10) nach einem der vorstehenden Ansprüche, wobei die Rückenstützvorrichtung (10) ein elastischer Stuhl ist.

12. Rückenstützvorrichtung (10) nach Anspruch 1, wobei der Verschluss an die elastischen Kniegurte (24, 26) an einer Stelle in der Mitte entlang ihrer Länge zwischen dem ersten (28, 32) und dem zweiten (30, 34) Ende gekoppelt ist.

## Revendications

1. Dispositif de soutien dorsal (10) pour soutenir le bas du dos d'un utilisateur, comprenant :
un élément de soutien dorsal à double extrémité (12) adapté pour fournir le soutien lombaire au bas du dos de l'utilisateur, dans lequel le soutien dorsal comprend une première extrémité (14) et une seconde extrémité (16) et une longueur entre la première et la seconde extrémité ;
une pluralité de sangles de genou élastiques (24, 26) s'étendant à partir des extrémités de l'élément de soutien dorsal, chacune de ladite pluralité de sangles de genou élastiques comprenant une première extrémité (28, 32) et une seconde extrémité (30, 34), les deux couplées à ou formées de manière solidaire avec une extrémité de l'élément de soutien dorsal (12) et formant une boucle (36, 38) adaptée pour mettre en prise un genou de l'utilisateur, lorsque l'utilisateur est dans une position assise ;
un mécanisme d'ajustement (58, 60) pour ajuster sélectivement les longueurs de chacune des sangles de genou élastiques (24, 26) ; et
une fixation (40, 42) pour fixer les sangles de genou élastiques (24, 26) ensemble à un emplacement situé entre lesdites première et seconde extrémités des sangles de genou élastiques (24, 26) ; et
un mécanisme d'ajustement de soutien dorsal (86) pour ajuster la longueur de l'élément de soutien dorsal.

2. Dispositif de soutien dorsal (10) selon la revendication 1, dans lequel l'élément de soutien dorsal comprend un matériau en tissu souple.

3. Dispositif de soutien dorsal (10) selon la revendication 2, dans lequel le matériau en tissu souple comprend un sanglage élastique.

4. Dispositif de soutien dorsal (10) selon la revendication 3, dans lequel le sanglage élastique comprend une pluralité de couches de sanglage.

5. Dispositif de soutien dorsal (10) selon l'une quelconque des revendications précédentes, dans lequel ladite pluralité de sangles de genou élastiques (24, 26) comprennent chacune un sanglage élastique.

6. Dispositif de soutien dorsal (10) selon l'une quelconque des revendications précédentes, dans lequel le mécanisme d'ajustement comprend un crochet en tissu adapté pour mettre en prise une pièce de réception en tissu.

7. Dispositif de soutien dorsal (10) selon l'une quelconque des revendications précédentes, dans lequel le mécanisme d'ajustement de soutien dorsal (58, 60) comprend un crochet en tissu adapté pour mettre en prise un certain nombre de rabats de réception en tissu (62, 64).

8. Dispositif de soutien dorsal (108) selon l'une quelconque des revendications précédentes, dans lequel l'élément de soutien dorsal (110) définit en outre une poche intérieure (124) adaptée pour permettre au dispositif de soutien dorsal (108) d'être replié sur lui-même et rangé à l'intérieur de cette dernière.

9. Dispositif de soutien dorsal (108) selon l'une quelconque des revendications précédentes, dans lequel l'élément de soutien dorsal (110) comprend en outre un certain nombre de poignées de transport (130, 132).

10. Dispositif de soutien dorsal (108) selon la revendication 8, comprenant en outre un dispositif thérapeutique disposé à l'intérieur de la poche intérieure (124).

11. Dispositif de soutien dorsal (10) selon l'une quelconque des revendications précédentes, dans lequel le dispositif de soutien dorsal (10) est une chaise élastique.

12. Dispositif de soutien dorsal (10) selon la revendication 1, dans lequel la fixation est couplée aux sangles de genou élastiques (24, 26) à un emplacement à mi-chemin le long de leur longueur entre lesdites première (28, 32) et seconde (30, 34) extrémités.
